# EUROPEAN PATENT APPLICATION

(11) **EP 2 703 932 A1**
(43) Date of publication of application: **05.03.2014**
(21) Application number: 12006091.8
(22) Date of filing: 28.08.2012

(54) **Personal communication device for managing individual fitness training**

(71) Applicant: SimpliFlow GmbH, 1050 Vienna (AT)
(72) Inventor: The designation of the inventor has not yet been filed

(57) **Abstract**

Managing individual fitness training over a communications network comprising a mobile client device (1) such as a smartphone, a server (2) and a computer program. Upon performing a physical self-testing conducted by the mobile client device (1), the server (2) generates a training plan and provides it to the mobile client device (1). During the training, the mobile client device (1) acquires training data (e.g. via GPS, pedometer, accelerometer, gyroscope, camera), analyses the training, provides feedback to the athlete and supports social networking.

Optionally, the mobile client device (1) is communicatively connected to an on-body sensor (3) via short-range communications.

## Description

### Field

The invention relates to a mobile communication device such as a smartphone, a server and a computer program for managing individual fitness training over communications networks. Basically the invention provides for generating training plans, supervising and analysing the training as well as interacting with social networks.

### Background

Concepts are known to determine stress levels during physical exercises and/or advice persons to intensify/reduce workout pace e.g. based on heart rate measurements. Furthermore concepts are known to record physical parameters during workout and to provide respective statistics and charts.

### Summary

The present invention relates to managing individual fitness training comprising a personal mobile communication (or client) device such as a smartphone, being in communication with a server over a mobile communications network and/or the Internet. An athlete aiming at achieving a certain fitness level registers at the server via the mobile client. Upon registration, the server provides data to the mobile client which then instructs the athlete to perform a physical self-testing (e.g. set of standardised exercises) in order to determine the physical constitution of the athlete.

The thus received self-test parameters are sent to the server where they are thoroughly analysed (e.g. by means of a knowledge base). A result in terms of a score for each test discipline is determined and, based on the score values, a personalised training plan is generated. Finally the training plan (e.g. covering a time frame of several weeks) is communicated to the mobile client which then instructs/leads the user throughout the whole training. The training plan may be modified/adapted based on parameters collected during the individual exercises, or via intermediate self tests.

Test parameters are either provided to the mobile client manually (e.g. manual input of heart rate, time, energy intake) or automatically by the mobile client (e.g. by means of GPS, accelerometer, gyroscope) and/or separate on-body sensors.

Social networking is provided such that e.g. an athlete may search for other persons in the vicinity having a similar training plan (or score levels).

### Drawings

Fig. 1 illustrates an embodiment of the present invention.
Fig. 2 illustrates a method according to the present invention.

### Description

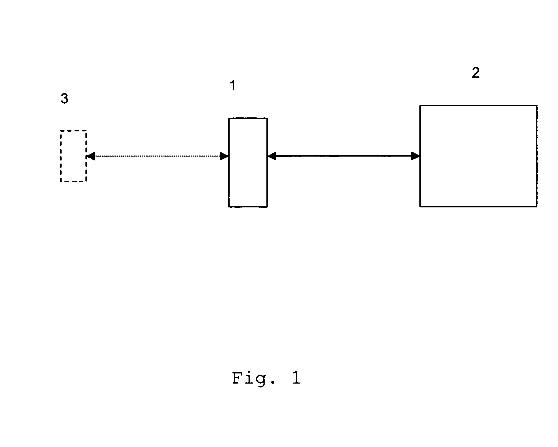

Fig. 1 shows a personal mobile client device 1 such as a smartphone or PDA. The client device is communicating with at least one server 2 over a communications network such as a public (mobile) telecommunications network (e.g. PSTN, GSM) and/or the Internet. The server is connected to (or comprises) a knowledge base for fitness applications. Furthermore the system may also comprise one or more on-body (or wearable) sensors 3 which are arranged to communicate with the mobile client via one-way or two-way short-range communications (e.g. Infrared, Bluetooth).

Physiological parameters (e.g. electrocardiographic data, heart rate) and other parameters such as acceleration, time, distance/pedometer may be determined by the on-body sensor(s) and/or the mobile device depending on the respective capabilities. Furthermore parameters such as weight, gender and age may be provided to the mobile client manually.

Essentially the mobile device acts as a training coach for the athlete and provides for monitoring, teaching, measuring, analysis and social networking.

In this regard, the mobile client device provides relevant training information (e.g. exercise type, starting time, duration, intensity, how to correctly perform exercise) in a multimedia-based form (i.e. text, audio, video, image, animation, interactivity) to the athlete. As an example the speed of playing animations may be based on a required intensity level. Alternatively vibrations, verbal announcements or audio/visual effects on the mobile device may indicate the required intensity.

Furthermore, the mobile client collects any relevant data of the athlete before, while and after exercising. This way the mobile device monitors the exercise and provides haptic or multimedia-based feedback to the athlete. As an example when it is determined (e.g. via GPS or pedometer) that the performance level (e.g. walking/running speed) is too low, respective feedback is provided. Video analysis further allows to control proper execution of the exercises. Thereby a video taken by the mobile client may be sent to the server for analysis. A (basic) analysis may even be provided by the mobile client itself.

The optional on-body sensor 3 provides additional information to the mobile client via short-range communication. It may comprise GPS, accelerometer, pedometer, gyroscope and/or pulse counting assembly to measure a variety of workout parameters. Depending on the workout the device may be fixed on different regions of a body (e.g. wrist, chest, leg, hip). It therefore comprises an adjustable fixing mechanism such as a belt, a hook, a loop fastener or an adhesive layer.

Furthermore when an athlete uses a device from a different manufacturer (e.g. heart rate monitor) the on-body sensor is arranged to collect data provided by that device. Thus the sensor allows to integrate other fitness related devices so that many physiological parameters may be taken into account by the system. Thus in an alternative version, the on-body sensor may solely comprise an interface for gathering data from other devices.

In the event that the sensor is not within communication distance with the mobile client (e.g. mobile client device is switched-off or not carried while running), the on-body sensor comprises a data storage for intermediately storing the collected data and transferring it to the mobile client at a later time.

An interface (e.g. display, tweet, speaker) for instantaneous audio/optical/haptic/tactile feedback is optionally provided thereby guiding/coaching the user during a workout session (e.g. different vibrations/forces when intensity is too high/low). Therefore the sensor is arranged to receive respective data from the mobile client device and/or generate such data locally (e.g. processing training data for basic analysis and feedback).

It is to be noted that all or part of the functionality mentioned in this context (e.g. data collection, feedback to athlete) can be implemented in the mobile client device and/or the on-body sensor(s). Furthermore functionality provided by other fitness-related devices is integrated thereby allowing a flexible configuration and data acquisition.

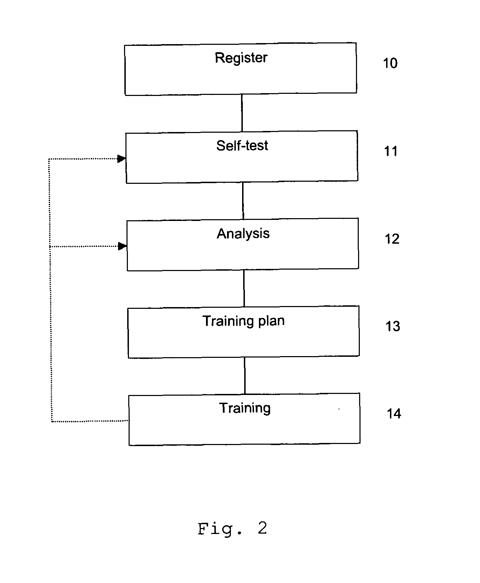

Fig. 2 illustrates the inventive method in a general form.

In step 10, a user registers his mobile client device over the communications network in the server in order to qualify for the service (e.g. sending an electronic message via the mobile client to the server to request a registration form or accessing a respective Internet page). Then the server provides a fitness testing routine (or application) to the mobile client (step 11). This fitness test comprises a basic set of standardised exercises in various disciplines (e.g. jumping power, endurance, speed) and a set of instructions of how to perform each exercise. It is primarily intended to assess the physical constitution of the user at the present time (self-testing). Typically this self-testing routine is loaded into the mobile device and then started locally. Alternatively it may also be started remotely at the server. The self-test is initiated e.g. by the athlete activating a software button on the touch screen (or launching the self-test routine). Performing the self-test is supported by the mobile client in that instructions of the respective exercises (including how to correctly perform them) are provided to the athlete (e.g. video, animation). Furthermore collecting the relevant result(s) is automated/supported as far as possible, depending on the available hardware (e.g. starting/stopping a timer via voice commands).

When the self-test is finished, the mobile client sends the collected data (e.g. also including parameters such as weight, gender and age) to the server for analysis (step 12). Thereby the server may comprise/consult a knowledge base to determine a score (e.g. 1-5) for each of the standard exercises.

Based on these scores, a training plan is generated (step 13). This plan may consist of a huge set of different exercises (up to several hundreds) to be performed over a period of up to several weeks and/or months. In addition the plan might also be sent (or "channeled") to a medical/fitness specialist/coach for modification(s) and/or approval (e.g. automatically sending an electronic message such as an email). Furthermore personal user preferences (e.g. time of day, season, weather, constraints/limitations due to user location and/or calendar) may be considered.

It is contemplated that the scores and/or training plans may be calculated (or arranged) by an adaptive system such as a self-learning expert system.

For each exercise the system holds information about which muscles (or muscle groups) are activated.

Eventually this may also be complemented by an intensity level. Thus when an athlete performs a certain exercise, the system knows which muscle is involved at what intensity levels. Information to that regard may be communicated to the athlete at any time e.g. by displaying a human model showing the activated muscles for a particular exercise, a training session or even the whole training plan. Different colours may be applied to reflect the respective and/or accumulated intensity level. In particular when an athlete arranges his training manually (e.g. entering a mode for manually selecting exercises to compose a training plan), this feature is advantageously in order to identify and avoid overloaded/overstrained muscles.

In any case, the training plan is then communicated to the mobile client which supports the user in putting the training plan into action (step 14). A live connection to a personal coach or video analysis is optionally in order to guarantee a proper workout.

Results, collected manually and/or automatically by means of the mobile client and/or the sensor(s), are sent to the server in a timely manner. This allows to continuously re-calculate the user's fitness level (e.g. scores). As a further aspect, it is contemplated to feed this data into a social network such that e.g. a user may search for other athletes with a similar fitness level or training plan.

During the training (e.g. after an exercise has been finished), the user may optionally initiate a further self-test (or analysis) in order to receive an updated training plan from the server (see arrows in Fig. 2).

Apart from generally managing training such as described above, the system is further arranged to support an athlete in many ways before, during and after workout sessions.

With regard to supporting an athlete prior to a training session, notifications of an upcoming exercise are generated in advance (e.g. reminder). One possibility to do so, is to integrate the training plan (received from the server) into a calendar application of the mobile client, thereby eventually utilising an already available/installed applications. This also allows to coordinate the training plan with other responsibilities (e.g. private, business). As an example, workout lessons shortly before or after an important business meeting (or during working hours in general) may be avoided. Optionally training sessions may be started manually when the conditions (e.g. time, place, temperature) are appropriate. Depending on where the meeting is to take place (e.g. geographical region) lessons may even be rescheduled for another day (e.g. either one day before or after the meeting) and/or training facilities (e.g. fitness center) may be arranged/booked accordingly.

When an athlete travels to a new location (e.g. business trip) the system helps to find (and eventually book) proper training facilities (e.g. running track, fitness studio).

Other conditions/parameters that may influence the training plan are weather, location, environmental data (e.g. atmospheric ozone) and traffic. These parameters are determined by the mobile client (e.g. via Internet search) and checked against the calendar/training plan. When bad weather conditions(e.g. rain, storm) and/or environmental risks (e.g. heavy ozone concentration) are to be expected/predicted for a given time and location, training sessions might be rescheduled (e.g. earlier in the morning; relocated/moved to indoor location). Alternatively heavy exercises might be replaced by light ones and/or the intensity level may be reduced accordingly.

In a similar way, training might be rescheduled when the training location would be difficult to be reached (e.g. traffic jams, delays with regard to public transportation such as train, bus and underground).

When a session is missed, the system allows to arrange (e.g. finding a track, booking a gym or facility) for a proper substitution.

With regard to social networking the concept provides for finding other athletes with similar fitness levels and/or training plans. By means of e.g. matching calendars and/or training plans of registered users, groups might be formed so that these athletes are encouraged to exercise together. The connection to the social network is provided by the mobile client device and/or the server.

Furthermore the concept supports to form other groups having similar or particular characteristics such as e.g. training plan, fitness level, group membership (gym membership) or friends in order to exercise together (or even to compete with each other).

During a workout session the mobile client collects any data that allows to monitor the exercise and/or to provide feedback to the athlete. Thereby the type of data (e.g. motional/physiological data) and the amount of data depend on the capabilities of the involved hardware (i.e. mobile client device, optional on-body sensor/s and other fitness related devices).

As an example when the mobile client determines by means of video analysis that an exercise is not performed properly, a corresponding animation can be provided to the athlete (e.g. demonstrating the correct exercise). Furthermore the speed of the animation may be adapted to the required intensity level.

A basic analysis may be provided by the mobile client or even the sensor/s while a further analysis is supported by the server via a respective data communication (e.g. live streaming).

As a further feature the provision of real-time data to a community is contemplated (e.g. picture/video, map location, running speed).

Feedback to the athlete is provided by the mobile client and/or the optional sensor/s.

Hardware components - installed in the mobile client or the wearable sensor(s) - are provided to perform various measurements and/or analysis such as:
- time measurements: e.g. by means of a real-time clock
- velocity/speed/distance: e.g. by means of GPS, pedometer (together with time measurement)
- movements/motion analysis: based on optical motion tracking/capturing by means of optical sender and receiver such as e.g. (infrared) LED and (infrared) camera
- kinematic analysis: e.g. by means of accelerometer, pedometer, gyroscope for measuring kinematic quantities (e.g. velocity/speed, acceleration, rotation, position, orientation, torque, angular momentum and other forces)
- physiological parameters: e.g. heart rate, pulse, burned energy/calories

Of course, in order to obtain valuable results, at least some of the hardware components (e.g. accelerometer, gyroscope, optical sender) have to be attached to respective body parts (e.g. leg, arm).

When a workout session is finished, the mobile client device sends the collected data to the server for analysis, calculating results, providing statistics (e.g. logging and displaying running track), adjusting training plans and sharing/comparing results with others (e.g. social networking).

As an example, all exercises which the athlete has performed are saved in a respective user-profile. Daily, weekly, monthly and yearly training reports are generated and may also show the user which muscle groups and body parts he/she has activated in what intensity and how much time was spent during the training. Such historical and statistical data allow the user to determine his/her personal training progress.

As already noted above, the training plan is generated based on the analysis of the physical constitution (e.g. determined during the self-test or intermediate tests during the training) and other generic parameters such as age, gender and/or weight. In addition other medical/physiological data may also be taken into consideration. In particular injuries (e.g. athletic injuries such as tearing a muscle) and health-related problems (e.g. diseases, chill/cold) are important to be taken into account for the training. Various input options may be presented to the athlete to collect such information.

As an example, a graphical (e.g. two- or three-dimensional) human model is provided to support the athlete gathering such information. The model may display/illustrate the human anatomy including organs (e.g. kidney) and/or body parts (e.g. leg, shoulder) and/or the muscle structure so that the athlete simply selects an organ (or body part or muscle) and briefly describes the relevant medical condition/state. As an example, when a muscle hurts, the athlete selects this muscle (or muscle group) on the model and specifies the pain (e.g. mild, moderate, strong/sever) by means of a pre-defined mask and/or menu options for that muscle. Thus the training may be modified in order to reflect these conditions (e.g. temporarily reducing burden on that specific muscle).

It is in particular advantageous to provide an interactive or animated three-dimensional (3D) human model to support identifying problematic areas.

Possible implementations for animated models are the following:
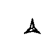 3D animated corpus for injury identification: Within this feature the user/athlete has the opportunity to detect the injury area. As an example he/she is clicking on his/her shoulder, mark the shoulder and save it as part of the body which causes pain. Then the user has the opportunity to select pain levels (e.g. scaling from 1 to 10, whereby 1 equals green and 10 equals dark red). The collected information is shown to the channels the athlete has defined (e.g. trainers, coaches, doctors, therapists). The involved parties have the opportunity to send the athlete/user exercises related to the injury. Injury related exercises are shown to the coaching staff based on the selection of the athlete/user on the 3D corpus. As soon as the athlete/user feels better, he can change the status of his shoulder (level of pain). Again this information is shared with his channels. All injuries are saved in the profile of the athlete so he/she can fall back to an injury history. The level of pain is deposited with textual information (e.g. slight pain, strong movement limitation, no active movement possible).
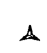 3D animated corpus for self made training planning: All available exercises in the knowledge management system are tagged to exercise specific muscle groups. When the user has access to the exercise portfolio (depending on the version he/she is using) then he/she can stick to a training plan via a professional training plan feature. Thereby the user selects exercises in order to compose a training. When the user is selecting for example a lot of exercises which activate a specific body part and/or muscle group then these parts are coloured (e.g. from white to red). If the body part or muscle group is shown red in the 3D animated corpus then the user knows that there is an overstraining of this specific body part.

All exercises which the athlete/user has chosen are saved in his profile. Periodic training reports are generated, showing the muscle groups and body parts he/she has activated in what intensity and how much time was spent during the training.

Possible colours are:
- White: No activation in the period
- Grey: Very low activation
- Green: adequate activation
- Orange: slight overstrain
- Red: Strong overstrain
   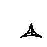 3D animated corpus for sports specific training analysis and training control:
      Based on the described model of a *self made training* all exercises which are chosen by the system and via the professional feature are tagged and saved, grouped by muscle groups and body parts. Furthermore the system automatically shows the athlete/user/trainer the sports specific relevant exercises. This means when a sports for example
   causes an increased shoulder, arm and lower back stress/load, then this is shown via the 3D corpus. The selected exercises of the system are automatically geared after the sports specific demand to avoid injuries. The professional feature also shows the trainer whether there is an overstrain of sensitive and/or specific body parts or whether there is a gap (or inconsistency) in the training structure.

## Claims

1. Method for managing individual fitness training with a mobile communication device, comprising:
a mobile client device (1) communicatively connected to a server (2),
the server (2) providing to the mobile client device (1) a fitness testing application for performing a physical self-test,
the mobile client device (1) conducting the physical self-test and collecting the results,
the mobile client device (1) forwarding the results to the server (2),
the server (2) analysing the results, determining a training plan and providing the training plan to the mobile client device (1), and
the mobile client device (1) conducting the training according to the received training plan.

2. Method according to claim 1 whereby the mobile client device (1) is communicatively connected to at least one on-body sensor (3) via short-range communications.

3. Method according to claim 2 whereby the mobile client device (1) and/or the at least one on-body sensor (3) comprises at least one of a real-time clock, a Global Positioning System GPS, a pedometer, an accelerometer, a gyroscope and a camera in order to measure kinematic quantities such as velocity, acceleration, rotation, position, orientation, torque and angular momentum and/or providing motion analysis.

4. Method according to claims 3 whereby the mobile client device (1) and/or the at least one on-body sensor (3) provide for multimedia and/or tactile feedback to a user.

5. Method according to claim 1 whereby the mobile client device (1) integrates the training plan into the calendar application of the user of the mobile client device and/or provides the training plan to a social network.

6. Method according to claim 1 whereby the mobile client device (1) collects data on the weather, environment and/or traffic and modifies the training plan in accordance to this data.

7. Method according to claim 1 whereby the mobile client device (1) displays an interactive graphical model of the human anatomy and the muscle structure for selecting organs, body parts or muscles, describing the relevant medical state and modifying the training plan based on the medical state.

8. Method according to claim 2 whereby the at least one on-body sensor (3) comprises a data storage for intermediately storing data when the communication with the mobile client device (1) is interrupted.

9. System for managing individual fitness training, comprising means adapted to perform the method of any of the preceding claim.

10. Computer program for managing individual fitness training, including executable instructions which, when executed, cause the system of claim 9 to perform the method according to any one of claims 1-8.
